Europäisches Patentamt

European Patent Office

Office européen des brevets

(11) Publication number: **0 177 195 B1**

## (12) EUROPEAN PATENT SPECIFICATION

(45) Date of publication of the patent specification:
**11.01.89**

(51) Int. Cl.⁴: **C07C 7/148**, B01J 21/10, C07C 6/12, C07C 2/66

(21) Application number: **85306337.8**

(22) Date of filing: **06.09.85**

(54) **Process for organic chloride removal from hydrocarbon feeds.**

(30) Priority: **24.09.84 US 653275**

(43) Date of publication of application:
**09.04.86 Bulletin 86/15**

(45) Publication of the grant of the patent:
**11.01.89 Bulletin 89/2**

(84) Designated Contracting States:
**BE DE FR GB IT NL**

(56) References cited:
**FR-A- 2 079 236**
**US-A- 2 328 707**

(73) Proprietor: **MOBIL OIL CORPORATION, 150 East 42nd Street, New York New York 10017(US)**

(72) Inventor: **McWilliams, John Paul, 117 S. American Street, Woodbury, NJ 08096(US)**
Inventor: **Nemet-Mavrodin, Margaret Iosif, 7 Lambert Lane, Robbinsville, NJ 08691(US)**
Inventor: **Sigal, Catherine Teague, 120 East Delaware Avenue, Pennington, NJ 08534(US)**
Inventor: **Wilson, Robert Currin, Jr, Deceased(US)**

(74) Representative: **Cooper, John Anthony et al, Mobil Court 3 Clements Inn, London WC2A 2EB(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to a method of removing organic chloride contaminants from hydrocarbon feedstocks. In particular, the invention is concerned with a method of protecting metal-containing zeolite catalysts against the deleterious effects of organic chlorides by use of a novel guard bed catalyst.

Hydrocarbons of natural or synthetic origin as generally available are often contaminated by the presence therein of impurities in substantial amounts, the removal of which is essential before such hydrocarbons can be efficiently employed as starting materials in processes of converting such hydrocarbons to more valuable derivatives thereof. In particular, chloride impurities in organically combined form may be of natural origin or have been introduced into the hydrocarbon charge during a chemical treating or processing operation.

The deleterious effects of halogens on catalytic function, in particular, on metal-containing hydrocarbon reforming catalysts is known and attempts have been made to treat the hydrocarbon charge for removal of the halogen components prior to reforming the charge. It has been found, for example, that organic chlorides can have a detrimental effect on the activity of the metal-containing reforming catalysts to such an extent that the catalyst loses its ability to promote the various individual reforming reactions. In addition, the catalyst may lose its ability to promote the desired reforming reactions such that the catalyst loses the desired selectivity for desired products.

Chloride contamination is particularly harmful in processes involving the disproportionation and alkylation of toluene to para-xylene and para-ethyltoluene over ZSM-5 zeolite catalysts which also contain magnesium. Such processes are disclosed in U.S. Patent Nos. 3 972 832; 4 034 053; 4 128 592; 4 137 195; 4 278 827 and 4 447 666. Chloride contamination of the toluene feedstock can alter the selectivity of the process so as to reduce the production of the more important para isomer.

As stated above, there have been previous attempts to remove organically combined halogens from hydrocarbon charge stocks. For example, U.S. Patent No. 2 413 871 discloses the removal of organically combined chlorine from hydrocarbons by subjecting the hydrocarbon to the action of a mixture of alumina in a suitably active form such as bauxite and quick lime under conditions such as to effect decomposition of the organic chloride compounds. U.S. Patent No. 2 481 300 discloses that contaminating amounts of impurities such as organically combined halogen can be removed substantially completely from hydrocarbons by contacting the hydrocarbons with a catalyst comprising active carbon in combination with an alkaline compound of an alkali and/or alkaline earth metal. Alkaline earth metals which are specifically mentioned include calcium, barium, and strontium.

U.S. Patent No. 2 951 804 discloses a method for the removal of acidic organic contaminants from reforming chargestocks by contacting the chargestock with an activated alumina having impregnated thereon an added base such as the hydroxides of alkali metals and alkaline earth metals, the preferred bases being the hydroxide of either sodium or potassium.

U.S. Patent No. 2 967 819 also discloses a method for protecting hydrocarbon reforming catalysts against the deleterious effect of halogen compounds by contacting the hydrocarbon charge with certain alkaline earth metal compounds prior to contacting the charge material with the metal-containing reforming catalyst. The charge is contacted with alkaline earth metal compounds such as calcium oxide, calcium sulfide, barium oxide, or barium sulfide or mixtures of two or more of these compounds. The compounds or mixture thereof are in the form of solid pellets or granules. In order to provide mechanical strength to the alkaline earth metal compound pellets, such compounds may be mixed prior to pelleting with an inert material such as alumina or magnesia.

U.S. Patent No. 3 278 266 discloses the separation of hydrogen halides from hydrocarbons by contacting the hydrocarbon charge with a diacid base deposited on a highly porous acid resistant support. Suitable diacid bases include magnesium, calcium, strontium, and barium in the form of their respective oxides or hydroxides. Generally, the alkaline earth base or the diacid deposit will be between 5 to 20% by weight although amounts as 35% and as low as 1% may be used.

U.S. Patent No. 3 898 153 discloses as a chloride scavenger the use of a caustic solution or a copper guard catalyst.

U.S. Patent No. 3 935 295 discloses a process for removing hydrogen chloride from a hydrocarbon stream by passing the hydrocarbon stream through a bed of dried and discrete absorbent particles consisting essentially of zinc oxide, a basic compound of calcium and an inert binder.

U.S.Patent No. 4 127 470 discloses a process of removing sulfur compounds from hydrocarbon feedstocks by contacting the feed with an alkali metal or alkaline earth metal including calcium, barium and magnesium oxides. Suitable supports can be employed such as alumina whereby the supported systems can be prepared by individually impregnating the support which is to be utilized with each reagent. Preferably, the alkaline earth metal compound is utilized as a support for the alkali metal compounds.

U.S. Patent 4 341 745 discloses the removal of acid gases from waste gases by contacting the gases with an absorbent which is a direct reaction product from a mixture of red mud and an alkaline earth metal hydroxide or aluminum oxide. Calcium is the only alkaline earth metal compound specifically disclosed in the patent.

Other methods of dehydrochlorinating (HCl elimination) are known. Note "Catalytic Reduction of Organic Chlorine Compounds in Hydrogen Stream by 50 percent Ni on Kieselguhr Catalysts", (Mokrousova et al, Kinet-Katal, 16 #3, 796–797 (1975); "Dehydrochlorination of Chloroalkanes on Solid Acids and Bases" [Mochida et al, J. Cat. 43 (1976); J. Org. Chem., 32 (1967), J. Org. Chem. 33 (1968)]; and SRI Report #102 "Disposal and Recovery of Waste Organo Chlorides" (1976).

US-A 2 328 707 discloses a method of removing chlorine from hydrocarbon mixtures by heating the mixture in the presence of Fuller's earth and magnesium oxide. The use of shaped particles comprising magnesium oxide and an inert binder is not disclosed in this patent.

FR-A 2 079 236 is directed to a process of purifying hydrocarbons containing organic chloride contaminants. The process comprises passing the hydrocarbon over a bed of particles constitued or coated by one or more of several reactive materials. However, the use of shaped particles comprising magnesium oxide and a binder is not described or suggested in this reference.

The present invention provides a method of removing organic chlorides from hydrocarbon feedstocks comprising passing the feedstock in contact with a catalyst formed by mixing magnesium oxide with a binder substantially inert with respect to the feedstock and shaping the mixture to produce shaped catalyst particles. By inert binder in this invention is meant a material which does not alter the composition of the hydrocarbon feedstock.

The use of the guard bed catalyst of the present invention prior to processing the hydrocarbon feedstock eliminates variations in catalyst activity and selectivity that has heretofore been the case when organic chlorides are present and allows relaxation of feed constraints as well as process parameters which required constant adjusting to maintain the quantitative production of desired products when chloride contamination deleteriously affected catalyst properties.

The guard bed catalyst of the present invention has important use in removing small amounts of organic chloride compounds from toluene feedstocks which are converted to para-xylene or para-ethyltoluene over magnesium-containing ZSM-5 catalysts. Although the chlorine impurities in commercially available toluene are extremely difficult to identify, they are believed to be primarily alkyl chlorides. In one toluene source, 1,1,1-trichloroethane has been identified as a particular chlorine-containing impurity.

As discussed above, alkaline earth metal oxides have known activity for organic chloride removal from hydrocarbon feeds. In particular, calcium oxide has been widely used to effect such removal. It has been found, however, that magnesium oxide when prepared according to this invention is more effective than calcium oxide in removing organic chloride contaminants from hydrocarbon feeds.

One disadvantage of using alkaline earth metal oxides, such as magnesium oxide, as a guard bed catalyst is that such materials are not readily processed into shaped particles having desirable physical characteristics such as adequate attrition resistance. Attrition-resistant particles of a uniform size are necessary to reduce pressure drop and flow distribution problems in a guard bed during operation. The most efficient method of obtaining such particles is by mixing magnesium oxide with a suitable binder prior to processing into shaped particles. Thus, in accordance with this invention, the attrition resistant problems of magnesium oxide particles are overcome by mixing magnesium oxide with an inert binder followed by controlled extrusion into, for example, 1/16-inch diameter cylinders, and subsequent calcination to produce uniform particles of good mechanical strength, relatively high surface area and excellent chloride removal activity. Preparation of catalyst particles in this controlled manner allows reproducible manufacture of a catalyst having the desired physical and catalytic properties.

The guard bed catalyst of the present invention is preferably in the form of an extrudate which comprises a binder able to be mixed with the active magnesium oxide adsorbent and extruded or pelletized therewith into the desired particle shape. The magnesium oxide component alone is not readily processed into shaped particles. By combining the magnesium oxide with a binder and forming shaped particles such as by the preferred method of extrusion, uniform particles can be continuously produced. Moreover, the guard bed catalyst in the form of an extrudate contains more magnesia than catalyst particles formed by methods of impregnating a support with a solution containing a magnesium compound and calcining to form the oxide. Thus, a catalyst extrudate can remove the organic chloride contaminants from the hydrocarbon feedstock more readily than impregnated supports. The guard bed catalyst particle of the present invention will contain at least about 50 wt.% magnesia, preferably, will contain over 60 wt.% and, more preferably, at least 70 wt.% of the active organic chloride removing agent. The remainder of the particle will be the binder.

The binder utilized to form the attrition-resistant guard bed catalyst particles must be inert with respect to conversion of the hydrocarbon feed being treated to remove organic chlorides, but the binder may exhibit dechlorination activity. The binder must also be readily extrudable, so that mixtures of magnesium oxide and binder can be extruded into particles of uniform size. The binder must be free of leachable chlorides. Examples of suitable binders include clay, silica, alumina, and silica-alumina. A preferred binder is Attapulgus clay which permits facile extrusions of guard bed catalyst particles containing levels of binder as low as 20 wt.%.

Attapulgite clay is found in the Georgia-Florida area of the United States, in India and in the Soviet Union. Attapulgite clay typically contains from about 70% to about 80% by weight of attapulgite, from about 10% to about 15% by weight of montmorillonite, sepiolite and other clays, from about 4% to about 8% by weight of quartz and from about 1% to about 5% by weight of calcite or dolomite.

Various refined versions of attapulgite clay are available from the Engelhard Minerals and Chemicals Corporation under the trade name of Attapulgus Clay. These refined products are beneficated by thermal activation, milling and screening. Non-clay fractions are removed during refinement such that the refined products may contain up to 85% to 90% by weight attapulgite. A typical chemical analysis for an Attapulgus Clay product would be approximately as follows:

| (Volatile-Free Basis) | |
|---|---|
| Silicon ($SiO_2$) | 68.0% |
| Aluminum ($Al_2O_3$) | 12.0% |
| Magnesium (MnO) | 10.5% |
| Iron ($Fe_2O_3$) | 5.0% |
| Calcium (CaO) | 1.7% |
| Phosphorus ($P_2O_5$) | 1.0% |
| Potassium ($K_2O$) | 1.0% |
| Titanium ($TiO_2$) | 0.7% |
| Trace Elements | 0.1% |
| | 100.0% |

The guard bed catalyst particles are produced by mulling magnesium oxide with the binder and water and extruding the mixture. The extruded particles are dried and calcined. By this method of forming the guard bed catalyst particles, the particles contain at least about 50 wt.% magnesium oxide and preferably, magnesia levels of at least 70 wt.%. In contrast, methods of forming catalyst particles by impregnating a support with a magnesium salt solution and calcining to yield the active component in the form of an oxide have been found to require multiple applications of the salt solutions and calcinations to provide a magnesium oxide loading of greater than 10%. The method of the present invention comprising intimately mixing the solid magnesium oxide with binder and extruding eliminates the need to work with large volumes of solution and is free of harmful emissions given off during calcination as opposed to the emissions formed by calcination of catalysts containing impregnated magnesium salts. More importantly, by forming the guard bed catalyst particles in accordance with the present invention, high magnesium oxide levels are achieved in a single extrusion. The high levels of active component in the guard bed catalyst particles result in high catalytic activity for the removal of organic chlorides and a high capacity for chloride retention.

The dechlorination of the hydrocarbon feedstock is achieved by contracting the feedstock with a bed of the extruded magnesium oxide/binder particles. Removal of the organic chlorides takes place at a temperature within the range of 177°C (350°F) to about 454°C (850°F) and space velocities varying from 1 to 35 weight hourly space velocity (WHSV). The hydrocarbon stream is preferably in vapor form. Preferably, lower temperatures are utilized such as 177°C (350°) to 316°C (600°F). The lower temperatures may necessitate lower space velocities ranging from about 1 to about 8 WHSV. It has been found that the magnesium oxide-containing guard bed catalyst can reduce the chloride content of a hydrocarbon feedstock to less than 1 ppmw and even less than 0.5 ppmw.

The following examples illustrate the present invention.

## Example 1

Various calcium oxide and magnesium oxide guard bed catalyst particles were formed and used to treat a stream of toluene containing 1,1,1-trichloroethane, which was used as a model organic chloride. Physical properties for the various calcined calcia/clay and magnesia/clay 1/16 inch diameter cylindrical extrudates are shown in Table 1. In all cases the binder was Attapulgus clay. The calcia/clay extrudates exhibited good crush strength averaging 11,082 gm/cm (62 pounds/inch) and relatively high surface areas averaging 56 m²/g. The magnesia/clay particles as formed in accordance with the present invention exhibited crush strengths of 9465 to 11,250 gm/cm (53 to 63 pounds/inch) and surface areas of 29 to 160 m²/g.

The catalyst particles of the present invention containing 70% magnesium oxide and 30% Attapulgus clay were formed by the following procedures:

60 grams of MagChem 700™ MgO from Martin Marietta Chemicals were mixed with 29.7 grams of Attagel and 84 grams of deionized water. Attagel 40 is a specially processed Attapulgus clay prepared by Engelhard. It is 80% solids with an average particle size of 0.14 . The water was added stepwise to the mixture of clay and magnesium oxide and the mixture was mulled for about 20 minutes. The measured percent solids of the mull mixture was 47%. The mixture was extruded into 0.16 cm (1/16-inch) diameter cylinders using a 2.54 cm (one-inch) diameter barrel auger extruder. The extruded particles were dried at 121°C (250°F) and calcined in air for four hours at a temperature of 538°C (1000°F).

800 g of MCB (Matheson Coleman Bell) reagent grade MgO were mixed in a muller with 396 g of Attagel 40. 1220 g of deionized water were then added stepwise to the mixture of MgO and clay. The mixture was mulled 20 minutes and had a percent solids of 46.0%. Extrusion of the mixture into 0.16 cm (1/16 inch) diameter cylinders was carried out using a 5.08 cm (2 inch) diameter barrel auger extruder. The extruded particles were dried at 121°C (250°F) and calcined in air for four hours at a temperature of 538°C (1000°F).

Calcia/clay particles were formed in a similar manner to the magnesia/clay particles. As indicated in Table 1, two types of extruders were used in these preparations: a ram extruder or a 5.08 cm (2 inch) diameter barrel auger extruder.

### Table 1
#### Physical Properties of Alkaline Earth Oxide/Attapulgus Clay Extrudates
0.16 cm diameter cylinders; calcined 4 hr at 538°C in air

| Catalyst Composition | (1) 50% CaO/ 50% clay, ram extruded | (2) 70% CaO/ 30% clay auger extruded (5.08 cm barrel) | (3) 70% CaO/ 30% clay auger extruded (5.08 cm barrel) | (4) 80% CaO/ 20% clay ram extruded | (5) 70% MgO/ 30% clay auger extruded (2.54 cm barrel) | (6) 70% MgO/ 30% clay auger extruded (5.08 cm barrel) |
|---|---|---|---|---|---|---|
| % solids of extrusion (538°C) | 48 | 45 | 47 | 49 | 47 | 46 |
| Crush strength gm/cm | 11072 | 8929 | 1465 | 9465 | 9286 | 11251 |
| Real densyty g/cc | 2.722 | 3.083 | 3.001 | 2.934 | 3.149 | 3.262 |
| Partial density, g/cc | 0.848 | 0.859 | 0.886 | 1.057 | 0.910 | 0.820 |
| Pore volume cc/g | 0.812 | 0.839 | 0.795 | 0.605 | 0.781 | 0.913 |
| Ave. pore diameter angstroms | 541 | 589 | 513 | 526 | 1078 | 228 |
| Surface area $m^2/g$ | 60 | 57 | 62 | 46 | 29 | 160 |

Dechlorination activity was measured for some of the catalysts shown in Table 1. The results are shown in Table 2 as a function of temperature and space velocity.

### Table 2
### Dechlorination Activity of Alkaline Earth Oxide/Attapulgus Clay Extrudates
### Atmospheric Pressure, Toluene Spiked with 1,1,1-trichloroethane

| Catalyst | Temp °C | Toluene WHSV | Cl in (*) Feed (ppmw) | Cl in Toluene Effluent (ppw) (*) at TOS | | | Avg. Cl Removal (%) |
|---|---|---|---|---|---|---|---|
| | | | | 0.5 hr | 1.0 hr | 1.5 hr | |
| (1) | 440 | 34 | 30 | 1 | 1 | 1 | 97 |
| 50% CaO/50% clay | 200 | 5 | 30 | 1 | 1 | 1 | 97 |
| (2) | 440 | 34 | 25 | 1 (.7 hr) | 1 (1.3hr) | 1 (2 hr) | 96 |
| 70% CaO/30% clay | 200 | 5 | 25 | | 2 | 6 (2 hr) | 84 |
| (3) | 440 | 33 | 32 | 4 | 6 | 6 | 83 |
| 70% CaO/30% clay | 200 | 5 | 32 | 1 | 1 | 3 | 95 |
| (5) | 440 | 34 | 26 | 0.5 | (0.75 hr) | 0.5 | 98 |
| 70% MgO/30% clay | 320 | 5 | 26 | – | 0.5 | 0.5 (2 hr) | 98 |
| | 200 | 5 | 26 | – | 0.5 | 0.5 (2 hr) | 98 |
| 100% clay, 0.08 cm | 440 | 34 | 27 | 1 | 4 | 8 | 84 |
| extrude | 200 | 5 | 27 | 14 | 18 | 16 | 41 |

| Catalyst | Temp °C | Toluene WHSV | Cl in Feed (ppmw) | 0.75 | 1 | 1.5 | 2 | 2.25 | 3 | 4 | Avg. Cl Removal (%) |
|---|---|---|---|---|---|---|---|---|---|---|---|
| | 200 | 5 | 67 | – | 4 | – | 4 | – | 5 | 7 | 93 |
| (6) | 320 | 5 | 67 | – | 2 | -- | 1 | – | 1 | 1 | 98 |
| 70% MgO/30% clay | 365 | 34 | 73 | 22 | – | 11 | – | 13 | 10 | – | 81 |

(*) Chloride analysis by x-ray fluorescence.

As can be seen from Table 2, the magnesia/clay guard bed catalyst particle is the most active. A run with a 0.16 cm, 100% Attapulgas clay extrudate as seen from Table 2 showed some activity for chloride removal at higher temperatures, e.g., 440°C. In all the runs shown in Table 2, toluene conversion was minimal, being of the order of 0.1%.

### Example 2

Guard bed catalysts formed by impregnating a magnesium salt on a support and calcining to form magnesium oxide were compared with regard to decholorination activity to the magnesium oxide/clay extrudate formed in Example 1. These guard bed catalysts are shown in Table 3.

### Table 3
### Mg Loading on Chloride Guard Bed Catalysts

| Catalyst | Wt% Mg | No. of Impregnations (*) |
|---|---|---|
| 70% MgO/30% clay extrudate | 42.2 | none |
| MgO on activated alumina beads (5×8 mesh) | 3.2 | 1 |
| MgO on activated alumina pebbles (8×14 mesh) | 4.9 | 1 |
| MgO on alumina extrudate | 4.9 | 1 |
| MgO on silica-alumina beads | 2.4 | 1 |
| MgO on silica extrudates | 14.6 | 2 |

(*) A nearly saturated salt solution of 60±5 wt.% $Mg(NO3)_2 \cdot 6H_2O$ was used for each impregnation.

Table 4 illustrates the dechlorination activity of some of the magnesium-containing guard bed catalysts shown in Table 3.

## Table 4
### Dechlorination Activity Mg-Containing Guard Bed Catalysts
### Atmospheric Pressure, Toluene Feed

| Catalyst | Temp (°C) | Toluene WHSV | Cl in (*) Feed (ppmw) | Cl in Effluent at TOS (ppmw) | | | | |
|---|---|---|---|---|---|---|---|---|
| | | | | 0.5 hr | 1.0 hr | 1.5 hr | 2.0 hr | 3.0 hr |
| 70% MgO/30% clay extrudate | 440 | 34 | 26 | – | 0.5 (.75 hr) | 0.5 | – | – |
| | 320 | 5 | 26 | – | 0.5 | – | 0.5 | 0.5 |
| | 200 | 5 | 26 | – | 0.5 | – | 0.5 | – |
| 4.9% Mg on activated alumina pebbles | 430 | 5 | 22 | – | 2 | – | 4 | 2 |
| 14.6% Mg on silica extrudates | 440 | 5 | 40 | 1 | 1 | 1 | – | – |

As can be seen, even at the lower temperature or higher space velocity, the magnesis/clay extrudates were more active in removing the chlorine from the toluene stream. The increase in dechlorination activity can be attributed to the greater amount of magnesium oxide which is present in the guard bed catalyst particle. It is important to note that the high level of magnesium oxide present in the extruded particle cannot be attained by a single impregnation of a binder material.

## Claims

1. A method of removing organic chlorids from hydrocarbon feedstock which method comprises contacting the feedstock with a catalyst comprising magnesium oxide and a material substantially inert with respect to the feedstock, characterised in that the catalyst is formed by mixing the magnesium oxide with, as binder, the substantially inert material; and shaping the mixture to produce shaped catalyst particles.

2. A method according to claim 1 wherein the catalyst particles are formed by extruding the mixture of magnesium oxide and binder.

3. A method according to claim 1 or 2 wherein said shaped particles contain at least 50 wt.% of magnesium oxide.

4. A method according to any preceding claim wherein the inert binder is clay, silica, alumina or silica-alumina.

5. A method according to any preceding claim wherein said binder is Attapulgus clay.

6. A method according to any preceding claim wherein the removal of organic chlorides takes place at a temperature within the range of 177°C to 454°C and a weight hourly space velocity of 1 to 34.

7. A method according to any preceding claim wherein the feedstock is an aromatic feedstock which is thereafter contacted with a disproportionation or alkylation catalyst.

8. A method according to any preceding claim wherein the feedstock comprises toluene.

9. A method according to claim 7 or 8 wherein the disproportionation or alkylation catalyst comprises ZSM-5 containing a magnesium component.

## Patentansprüche

1. Verfahren zur Entfernung organischer Chloride aus Kohlenwasserstoffausgangsmaterialien, wobei dieses Verfahren den Kontakt dieses Ausgangsmaterials mit einem Katalysator umfaßt, der Magnesium und ein in bezug auf dieses Ausgangsmaterial im wesentlichen inertes Material umfaßt, dadurch gekennzeichnet, daß dieser Katalysator durch Vermischen von Magnesiumoxid mit dem im wesentlichen inerten Material als Bindemittel und Formen dieser Mischung gebildet sind, um geformte Katalysatorpartikel herzustellen.

2. Verfahren nach Anspruch 1, worin die Katalysatorpartikel durch Extrusion dieser Mischung von Magnesiumoxid und dem Bindemittel gebildet werden.

3. Verfahren nach Anspruch 1 oder 2, worin die geformten Partikel zumindest 50 Gew.-% Magnesiumoxid enthalten.

4. Verfahren nach einem der vorstehenden Ansprüche, worin das inerte Bindemittel Ton, Siliziumdioxid, Aluminiumoxid oder Siliziumdioxid/Aluminiumoxid ist.

5. Verfahren nach einem der vorstehenden Ansprüche, worin das Bindemittel Attapulgit-Ton ist.

6. Verfahren nach einem der vorstehenden Ansprüche, worin die Entfernung der organischen Chloride bei einer Temperatur im Bereich von 177°C bis 454°C und einer stündlichen Gewichts-Raum-Geschwindigkeit von 1 bis 34 stattfindet.

7

EP 0 177 195 B1

7. Verfahren nach einem der vorstehenden Ansprüche, worin das Ausgangsmaterial ein aromatisches Ausgangsmaterial ist, das danach mit einem Disproportionierungs- oder Alkylierungs-Katalysator in Kontakt gebracht wird.

8. Verfahren nach einem der vorstehenden Ansprüche, worin das Ausgangsmaterial Toluol umfaßt.

9. Verfahren nach einem der Ansprüche 1 bis 7 oder 8, worin der Disproportionierungs- oder Alkylierungs-Katalysator ZSM-5 umfaßt, der eine Magnesiumkomponente enthält.

**Revendications**

1. Un procédé d'élimination de chlorures organiques de charges hydrocarbonées, ce procédé consistant à mettre la charge au contact d'un catalyseur comprenant de l'oxyde de magnésium et un matériau sensiblement inerte vis-à-vis de la charge, caractérisé en ce que l'on forme le catalyseur en mélangeant l'oxyde de magnésium avec, à titre de liant, le matériau sensiblement inerte; et en ce que l'on conforme le mélange pour obtenir des particules de catalyseur conformées.

2. Un procédé selon la revendication 1, dans lequel on forme les particules du catalyseur par extrusion du mélange d'oxyde de magnésium et de liant.

3. Un procédé selon la revendication 1 ou 2, dans lequel ces particules conformées contiennent au moins 50% en poids d'oxyde de magnésium.

4. Un procédé selon l'une quelconque des revendications précédentes, dans lequel le liant inerte est l'argile, la silice, l'alumine ou un mélange silice-alumine.

5. Un procédé selon l'une quelconque des revendications précédentes, dans lequel ce liant est une argile d'Attapulgite.

6. Un procédé selon l'une quelconque des revendications précédentes, dans lequel l'élimination des chlorures organiques a lieu à une température comprise entre 177 et 450°C et à une vitesse spatiale horaire pondérale comprise entre 1 et 34.

7. Un procédé selon l'une quelconque des revendications précédentes, dans lequel la charge est une charge d'aromatiques que l'on met ultérieurement au contact d'un catalyseur de disproportionnation ou d'alkylation.

8. Un procédé selon l'une quelconque des revendications précédentes, dans lequel la charge comprend du toluène.

9. Un procédé selon la revendication 7 ou 8, dans lequel le catalyseur de disproportionnation ou d'alkylation comprend de la ZSM-5 contenant un dérivé du magnésium.